(19) Europäisches Patentamt
European Patent Office
Office européen des brevets

(11) Publication number: **0 043 247 B1**

(12) **EUROPEAN PATENT SPECIFICATION**

(45) Date of publication of patent specification: 01.02.84

(51) Int. Cl.³: C 07 C 103/52, G 01 N 33/56

(21) Application number: 81302874.3

(22) Date of filing: 25.06.81

# (54) Peptides for use in determining serum thymic factor.

(30) Priority: 25.06.80 JP 86952/80

(43) Date of publication of application: 06.01.82 Bulletin 82/1

(45) Publication of the grant of the patent: 01.02.84 Bulletin 84/5

(84) Designated Contracting States: CH DE FR GB LI NL SE

(56) References cited:
FR - A - 2 391 994
FR - A - 2 407 471

(73) Proprietor: KUREHA KAGAKU KOGYO KABUSHIKI KAISHA
9-11 Horidome-cho 1-chome Nihonbashi Chuo-ku
Tokyo (JP)

(72) Inventor: Yanaihara, Noboru
403-22 Kita Shizuoka-shi
Shizuoka-ken (JP)
Inventor: Konno, Koji
61, Sakai-Horinouchi Nakoso-machi
Iwaki-shi Fukushima-ken (JP)

(74) Representative: Myerscough, Philip Boyd et al,
J.A.Kemp & Co. 14, South Square Gray's Inn
London, WC1R 5EU (GB)

EP 0 043 247 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Peptides for use in determining serum thymic factor

This invention relates to peptide derivatives of tyrosine, which have physiological functions similar to those of so-called Serum Thymic Factor (hereinafter abbreviated as STF), to their production, to these peptides labelled with radioactive iodine and to the use of these radioactvely labelled peptides in the radioimmunoassay of STF.

With regard to the primary structure of STF, Jean-Fransois Bach *et al* reported in 1977 that an active ingredient extracted from swine serum showing activity as STF had the following structure:

pGlu-Ala-Lys-Ser-Gln-Gly-Gly-Ser-Asn-OH

[refer to J. Biol. Chem., 252 8045(1977)].

The symbols Ser, Gln, Gly, Asn, Tyr, Ala and Lys used herein represent the divalent amino acid residue formed by removing one hydrogen atom from the amino group and one hydroxyl group from the carboxyl group of the respective alpha- amino acids serine, glutamine, glycine, asparagine, tyrosine, alanine and lysine. For instance, Ser represents the divalent residue, —NH—CH($CH_2OH$)—CO—.

The thymus is an organ having a close relationship to antibody-production and cellular immunity. It is known that several peptides which are considered to be secreted by the thymus regulate immunological functions. The action of STF on thymix cells to increase the thymic cyclic AMP resulting in a contribution to the differentiation of thymic cells has been indicated by A. Astaldi *et al* [refer to Nature (London), *260* 713 (1977)]. Consequently STF is an interesting peptide as a factor secreted by the thymus and contained in serum.

Where the immuno-regulating action of STF is utilized clinically or for other purposes, it is necessary to accurately measure the concentration of STF in blood. Jean-Fransois Bach *et al* (refer to Bulletin de L'Institut Pasteur, 1978, 325) reported that the concentration of STF in blood is of the order of several $10^{-9}$kg/m$^3$ (pg/ml). Although it is possible to carry out a radioimmunoassay (RIA) to analyze substances present in such minute amounts, as can be seen from its amino acid-composition, STF does not contain an amino acid having aromatic ring(s) which can be labelled with radioactive iodine as in the case of histidine. Accordingly, in order to carry out RIA, it is necessary to introduce into the substance in advance of RIA a structure which can be easily radio-labelled. Moreover, although plenty of pure STF and radio-labelled STF is necessary to carry out RIA of STF, it is not profitable to collect large amounts of STF from animal blood and in a pure form when, for example, the amount of STF present in nature and the difficulty of purifying STF are considered.

FR—A—2391994 describes STF derivatives of the formula:

X-Gln-Gly-Gly-Y

in which Y represents -Ser-Asn and X represents Ser-, Lys-Ser-, Ala-Lys-Ser- or Glx-Ala-Lys-Ser-, Glx representing PyroGlu or Gln, and when X represents Glx-Ala-Lys-Ser- Y can also represent -Ser; as well as derivatives thereof in which 1 or 2 amino acids are modified but not such as to form STF itself.

FR—A—2407471 describes a method for the determination of the STF in a sample in which a competitive reaction is set up between the STF in the sample and a labelled STF and anti-STF antibody. STF derivatives as disclosed in FR—A—2391994 can be used to prepare the labelled STF or anti- STF antibody.

Deratives of tyrosine have now been synthesised which possess a similar chemical structure to STF and which can easily be labelled with radioactive iodine. These derivatives have a fairly high physiological activity similar to that of STF.

Accordingly, the present invention provides a peptide with a primary structure represented by the general formula (I):

X-Ser-Gln-Gly-Gly-Ser-Asn-Y (I)

wherein

(1) X represents H-Tyr-Gln-Ala-Lys and Y represents a hydroxyl group,

(2) X represents pGlu-Ala-Lys(N$^\varepsilon$-Tyr-H) and Y represents a hydroxyl group, or

(3) X represents pGlu-Ala-Lys and Y represents a Tyr-OH group, and

(a) -Lys(N$^\varepsilon$-Tyr-H) means that the amino group at the $\varepsilon$-position of the side chain of lysine is bonded to tryosine via a peptide bond, and (b) pGlu represents a group which has been formed by removal of a hydroxy group from the carboxy group of pyroglutamic acid.

These peptides can be produced by condensing the appropriate amino acid(s) and/or peptide(s) in order to obtain the peptide with the desired primary structure. Condensation can be effected in a conventional manner in a number of steps and suitably protecting various groups.

Peptides of formula (I) of the present invention are:

Compound No. 1: pGlu-Ala-Lys(N$^\varepsilon$-Tyr-H)-Ser-Gln-Gly-Gly-Ser-Asn-OH,

Compound No. 2: pGlu-Ala-Lys-Ser-Gln-Gly-Gly-Ser-Asn-Tyr-OH and
Compound No. 3: H-Tyr-Gln-Ala-Lys-Ser-Gln-Gly-Gly-Ser-Asn-OH.

The present invention also provides a preferred process for producing a peptide of formula (I) which comprises bonding a predetermined amino acid or peptide to the N-end of H-Asn-OH, H-Asn-Tyr-OH or a peptide of the appropriate primary structure and terminating in -Asn-OH or -Asn-Tyr-OH at its C-end without protecting the carboxyl group at the C-end of said H-Asn-OH, H-Asn-Tyr-OH or peptide terminating in -Asn-OH or -Asn-Tyr-OH at its C-end, and as desired elongating the resulting peptide chain from the N-end by the bonding of further predetermined amino acid(s) and/or peptide(s), to obtain the desired peptide.

Accordingly, the condensation method adopted for a synthesis, proceeding according to the preferred process of the invention, should be such that there are no problems due to the presence of a free carboxyl group. In the invention, the azide method, mixed acid anhydride method or active ester method is adopted.

On carrying out a condensation according to the preferred process of the present invention, an amino acid or a peptide having an unprotected C-terminal carboxyl group is used. However, it is necessary to protect the amino group at the N-end or in the side chain of the component providing the carboxyl group involved in the condensation reaction. Any protecting group utilized in ordinary peptide-synthesis for protecting amino groups can be used, for instance a benzyloxycarbonyl group, methoxybenzyloxycarbonyl group, chlorobenzyloxy-carbonyl group, nitrobenzyloxycarbonyl group, phenylazobenzyloxycarbonyl group, methoxyphenylazobenzyloxycarbonyl group, t-butoxycarbonyl group, 1-(1-biphenylyl)-isopropoxycarbonyl group, 1-(1-isopropyl-2-methyl)-propoxycarbonyl group, formyl group, acetyl group, benzoyl group, phthalyl group, tosyl group or 2-nitrophenylsulphenyl group.

Ordinary methods can be used to remove the protecting group after synthesis. For instance, a benzyloxycarbonyl group can be removed by catalytic hydrogenation in a solution of methanol at ambient temperature and pressure in the presence of a Pd catalyst, with a preferable result because of the easy treatment after the removal of the protecting group. The intentional addition of an acidic substance in an equimolecular amount or more to the peptide in the solvent frequently promotes the reaction. As for methoxybenzyloxycarbonyl-, chlorobenzyloxycarbonyl-, phenylazobenzyloxycarbonyl- and methoxyphenylazobenzyloxycarbonyl groups, a similar procedure can be taken.

In the other hand, a t-butoxycarbonyl group can be removed by an acidic reagent, for instance hydrogen chloride in acetic acid or in ethyl acetate and trifluoroacetic acid, etc. 1-(1-Bi-phenyl)-isopropoxycarbonyl-, 1-(1-isopropyl-2-methyl)propoxycarbonyl- and 2-nitrophenylsulphenyl groups are also removed by a similar reagent. Since a tosyl group is a relatively stable protecting group and cannot be removed by catalytic hydrogenation or acid-treatment, it is preferably removed by treatment with metallic sodium in liquid ammonia.

As has been stated, the conditions for removing protecting groups are the same or different depending on the kinds of protecting group. It is possible to remove only the desired group while maintaining other protecting group by suitably selecting these conditions.

In the present invention, the benzyloxycarbonyl-, t-butoxycarbonyl- and tosyl groups are the most convenient groups for protection of an amino group. By using one of these groups, pure peptide can be synthesized in a favorable yield. The thus obtained peptide is purified by ordinary procedures, for instance recrystallization, extraction or chromatographical separation. We have purified the synthesized peptide by a combination of several purification procedures until a single spot is obtained on a thin layer chromatogram by using a plurality of developing solvents, and have carried out identification of the product by, for example, amino acid analysis, infrared absorption spectroscopy or nuclear magnetic resonance spectroscopy.

The peptides of the present invention are novel peptides having physiological activities similar to those of STF. Since they are easily labelled with radioactive iodine, they are preferably effective as radio-labelled peptides when RIA is carried out on STF. Labelling with radioactive iodine can be accomplished by a conventional method.

The determination of STF-like activity of a peptide of the present invention was carried out by the Rosette test adopted by Jean-Francois Bach *et al* [refer to Journal Biol. Chem., *252*, 8040(1977), and Japanese Patent Application Laying Open No. 16425/79, published on Feb. 7, 1979].

Some of the physical properties of the three peptides of the present invention are shown below:

(a) pGlu-Ala-Lys($N^\epsilon$-Tyr-H)-Ser-Gln-Gly-Gly-Ser-Asn-OH (Compound No. 1) Peaks in thin layer chromatogram:
$Rf_1$ of 0.45; $Rf_2$ of 0.25 and $Rf_3$ of 0.54.
Specific rotatory power:
$[\alpha]_D^{24} = -49°$ (c = 0.308 in water)
Amino acid composition (In the amino acid compositions throughout this specification Gln shows as Glu due to hydrolysis occurring when the amino acids are determined):
$NH_3$—2.05; Lys—0.96; Tyr—0.93; Ala—0.98; Gly—1.97; Glu—2.04; Ser—1.79 and Asp—1.00.

(b) pGlu-Ala-Lys-Ser-Gln-Gly-Gly-Ser-Asn-Tyr-OH (Compound No. 2)
Peaks in thin layer chromatogram:
$Rf_1$ of 0.41; $Rf_2$ of 0.12 and $Rf_3$ of 0.30.

Specific rotatory power:
$[\alpha]_D^{24} = -55.5°$ (c = 0.263 in water)
Amino acid composition:
$NH_3$—3.51; Lys—1.04; Tyr—0.89; Ala—1.07; Gly—2.06; Glu—205; Ser—1.90 and Asp—1.00.

(c) H-Tyr-Gln-Ala-Lys-Ser-Gln-Gly-Gly-Ser-Asn-OH (Compound No. 3)
Peaks in thin layer chromatogram:
$Rf_1$ of 0.29; $Rf_2$ of 0.10 and $Rf_3$ of 0.26.
Specific rotatory power:
$[\alpha]_D^{24} = -44.1°$ (c = 0.279 in water)
Amino acid composition:
$NH_3$—3.59; Lys—1.06; Tyr—0.92; Ala—0.97; Gly—1.97; Glu—2.04; Ser—1.82 and Asp—1.00.

Notes:
i) The developing solvents for the thin layer chromatography were:
for $Rf_1$: a mixture of ethanol and aqueous 1M $CH_3COONH_4$ solution at a ratio of 7:3.
for $Rf_2$: a 2:2:1 mixture of chloroform, methanol and concentrated aqueous ammoniacal solution.
for $Rf_3$: a 1:2:1 mixture of chloroform, methanol and concentrated aqueous ammoniacal solution.
ii) The figures for the amino acid composition show the relative mol ratio of each amino acid when the molar amount of Asp is 1.00.

The following Examples illustrate the present invention.

Example 1: Synthesis of Compound No. 1

(1) Synthesis of H-Ser-Asn-OH

$2.53 \times 10^{-2}$Kg (25.3 g) of benzyloxycarbonylserine hydrazide was dispersed in suspension in $2 \times 10^{-4}$ $m^3$ (200 ml) of dimethylformamide. While keeping the suspension at −35 to −30°C, $5 \times 10^{-5} m^3$ (50 ml) of 6N hydrogen chloride solution in dioxane was added dropwise to the suspension. Then while keeping the suspension at −30 to −25°C, $1.5 \times 10^{-5}$ $m^3$ (15 ml) of isoamyl nitrate was added dropwise to the suspension. After keeping the mixture for 5 min at −20 to −15°C, the mixture was cooled to −35 to −30°C, and $4.2 \times 10^{-5} m^3$ (42 ml) of triethylamine was added dropwise to the mixture. The thus obtained slurry-like material was added to a mixture composed of $1.5 \times 10^{-2}$ kg (15.0 g) of asparagine hydrate, $5 \times 10^{-5} m^3$ (50 ml) of dimethylformamide, $2 \times 10^{-4} m^3$ (200 ml) of water and $2.8 \times 10^{-5} m^3$ (28 ml) of triethylamine. This mixture was stirred for one hour at −10 to 0°C. Stirring was then continued overnight at room temperature. After drying the mixture almost to dryness at a reduced pressure, $10^{-4} m^3$ (100 ml) of water was added to dissolve the semisolid. After adjusting the pH of the aqueous solution to 7.0 by triethylamine, the solution was extracted with ethyl acetate, and the aqueous layer was adjusted pH 4.5 with acetic acid and extracted with n-butanol. The substance obtained by evaporating off the n-butanol under reduced pressure from the butanol-extract was dissolved in a solvent mixture of $1.5 \times 10^{-4} m^3$ (150 ml) of methanol, $1.5 \times 10^{-4} m^3$ (150 ml) of water and $5 \times 10^{-6} m^3$ (5 ml) of acetic acid. The solution was subjected to catalytic hydrogenation at ambient temperature and pressure in the presence of a catalyst of $3 \times 10^{-3}$ kg (3 g) of 5% palladium on carbon for 5 hours. Then the reaction mixture was filtered. The filtrate was condensed under reduced pressure to obtain a product which was recrystallized from a mixture of water and ethanol in a yield of 45%. This product, H-Ser-Asn-OH, melted at 155 to 157°C.

(2) Synthesis of H-Gly-Gly-Ser-Asn-OH

H-Ser-Asn-OH prepared in (1) and benzyloxycarbonyl-Gly-Gly-$NHNH_2$ were condensed according to the azide method. The reaction mixture was extracted with n-butanol to separate benzyloxycarbonyl-Gly-Gly-Ser-Asn-OH. The product was subjected to catalytic hydrogenation in the presence of 5% palladium on carbon to obtain H-Gly-Gly-Ser-Asn-OH melting at 219°C with decomposition in a yield of 63%.

(3) Synthesis of H-Lys($N^\epsilon$-tosyl)-Ser-Gln-Gly-Gly-Ser-Asn-OH

H-Gly-Gly-Ser-Asn-OH obtained in (2) and benzyloxycarbonyl-Lys($N^\epsilon$-tosyl)-Ser-Gln-NH.$NH_2$ were condensed according to the azide method to obtain benzyloxycarbonyl-Lys($N^\epsilon$-tosyl)-Ser-Gln-Gly-Gly-Ser-Asn-OH. This product was subjected to catalytic hydrogenation in the presence of 5% palladium on carbon to obtain the desired peptide, H-Lys($N^\epsilon$-tosyl)-Ser-Gln-Gly-Gly-Ser-Asn-OH. After recrystallization from a mixture of water and ethanol, the yield was 33%. The amino acid composition of the product was: $NH_3$—2.24; Lys—0.28; Gly—2.08; Glu—0.99; Ser—1.78 and Asp—1.00.

(4) Synthesis of H-Ala-Lys($N^\epsilon$-tosyl)-Ser-Gln-Gly-Gly-Ser-Asn-OH

$2.7 \times 10^{-3}$ Kg (2.7 g) of H-Lys($N^\epsilon$-tosyl)-Ser-Gln-Gly-Gly-Ser-Asn-OH prepared in (3) was dissolved in $3 \times 10^{-5} m^3$ (30 ml) of dimethylsulfoxide. $5 \times 10^{-7} M^3$ (0.5 ml) of triethylamine was added to the solution. $2 \times 10^{-5} M^3$ (20 ml) of dimethylsulfoxide containing $1.56 \times 10^{-3}$ kg (1.56 g) of benzyloxycarbonyl-alanine N-hydroxysuccinimide ester was added to this mixture. The mixture was stirred overnight at room temperature. Then, after evaporating off the solvent from the reaction mixture under reduced pressure, the residue was washed thoroughly with chloroform and dried. The dried

residue was suspended in $10^{-4}m^3$ (100 ml) of water. The pH of this suspension was adjusted to 8.5 by adding triethylamine and the insoluble matter was then removed by filtration. The filtrate was made acidic with citric acid, and a gel-like product was obtained. After filtering off the gel-like product, washing the residue with ethanol and then ethyl acetate, and drying the washed residue, the thus obtained benzyloxycarbonyl-Ala-Lys($N^\varepsilon$-tosyl)-Ser-Gln-Gly-Gly-Ser-Asn-OH was subjected to catalytic hydrogenation in the presence of 5% palladium on carbon to obtain the desired peptide, H-Ala-Lys($N^\varepsilon$-tosyl)-Ser-Gln-Gly-Gly-Ser-Asn-OH. This crude product was purified by using Sephadex (Registered Trade Mark) G—10 while using an aqueous 1M acetic acid solution as an eluant. The amino acid composition of the purified product was: $NH_3$—2.10; Ala—1.01; Lys—0.35; Gly-2.02; Glu—0.99; Ser—1.70 and Asp—1.00.

(5) Synthesis of pGlu-Ala-Lys-Ser-Gln-Gly-Gly-Ser-Asn-OH

$9 \times 10^{-4}$ Kg (0.9 g) of H-Ala-Lys($N^\varepsilon$-tosyl)-Ser-Gln-Gly-Gly-Ser-Asn-OH obtained in (4) was dissolved in a mixture of $2.5 \times 10^{-5}m^3$ (25 ml) of dimethylformamide and $1.5 \times 10^{-5}m^3$ (15 ml) of water. After addition of $10^{-7}m^3$ (0.1 ml) of triethylamine to the solution, benzyloxycarbonyl-pGlu N-hydroxysuccinimide ester was added and the mixture was stirred overnight. Then the solvent was evaporated off from the reaction mixture and the residue was dissolved in water. After making the mixture acidic by addition of citric acid, the thus formed gel-like product was collected by filtration. This product was washed with a dilute aqueous citric acid solution, water, ethanol and ethyl acetate in that order. The product was then dried, followed by recrystallization from a mixture of dimethylformamide and ethanol. After subjecting the crystals to catalytic hydrogenation with 5% palladium on carbon, the product was dissolved in liquid ammonia and the protecting group of the product was removed by addition of metallic sodium. The thus treated product was subjected to an anion-exchange resin (IRA 411) and then purified by Biogel P—2 using water as an eluant. The yield of the desired purified product was 42%. The amino acid composition of this product was: $NH_3$—2.11; Ala—0.99; Lys—1.04; Gly—2.03: Glu—1.99; Ser—1.78 and Asp—1.00.

(6) The last step: Synthesis of Compound No. 1, pGlu-Ala-Lys($N^\varepsilon$-Tyr-H)-Ser-Gln-Gly-Gly-Ser-Asn-OH

$4.86 \times 10^{-4}$Kg (486 mg) of pGlu-Ala-Lys-Ser-Gln-Gly-Gly-Ser-Asn-OH obtained in (5) was dissolved in a mixture of $6 \times 10^{-6}m^3$ (6 ml) of dimethylformamide and $4 \times 10^{-6}m^3$ (4 ml) of water. After addition of $1.4 \times 10^{-7}m^3$ (0.14 ml) of triethylamine to the solution, $10^{-5}m^3$ (10 ml) of dimethylformamide containing $3 \times 10^{-4}$ kg (0.3 g) of benzyloxycarbonyltyrosine N-hydroxysuccinimide ester was added to the mixture. This was stirred overnight.

After evaporating off the solvent from the reaction mixture under reduced pressure and adding a few drops of acetic acid to the residue, ether was added to deposit crystals. After washing the crystals with ethyl acetate, the crystals were dried and dissolved in aqueous 50% acetic acid. This solution was subjected to catalytic hydrogenation in the presence of $3 \times 10^{-4}$ kg (0.3 g) of palladium black. After the reaction was over, the reaction mixture was filtered. The filtrate was condensed under reduced pressure. The residue was dissolved in $6 \times 10^{-6}m^3$ (6 ml) of water. This solution was subjected to chromatography on Biogel P—2, using water as an eluant, to purify the product. The yield of the desired product was 35%, the amino acid composition of the product being:
$NH_3$—3.00; Ala—0.98; Lys—0.98; Gly—2.00; Glu—2.06; Ser—1.89; Tyr—1.02; Asp—1.00.

## Example 2: Synthesis of Compound No. 2:

(1) Synthesis of H-Ser-Asn-Tyr-OH

$2.77 \times 10^{-2}$ Kg (27.7 g) of benzyloxycarbonyl-Asn-OH and $4.61 \times 10^{-2}$ kg (46.1 g) of H-Tyr-O.benzyl. Tosyl-OH were dissolved in $5 \times 10^{-4}m^3$ (500 ml) of dimethylformamide. $1.86 \times 10^{-2}$ Kg (18.6 g) of N-hydroxy-5-norbonene-2,3-dicarboxyimide, $1.46 \times 10^{-5}m^3$ (14.6 ml) of triethylamine and $2.14 \times 10^{-2}$kg (21.4 g) of dicyclohexylcarbodiimide were added at −5°C to this solution and the mixture was stirred overnight. After the reaction was over, the reaction mixture was filtered. The filtrate was condensed under reduced pressure to a viscous liquid. On adding water to the viscous liquid, crystals deposited. The crystals were washed with aqueous saturated solution of sodium hydrogen carbonate, dilute hydrochloric acid, ethanol and ether, in that order, and then dried. The thus obtained crystals were dissolved in a mixed solvent of $2.6 \times 10^{-4}m^3$ (260 ml) of dimethylformamide, $3.5 \times 10^{-5}m^3$ (35 ml) of water and $6.5 \times 10^{-5}m^3$ (65 ml) of acetic acid and subjected to catalytic hydrogenation in the presence of $10^{-3}$kg (1 g) of palladium black at ambeint temperature and pressure for 7 hours.

Then, the reaction mixture was filtered. The filtrate was condensed under reduced pressure. Water was added to the condensate and the matter insoluble in water was filtered off. The filtrate was condensed under reduced pressure to obtain crystals, which were recrystallized from a mixture of water and ethanol to obtain H—Asn-Tyr-OH.$CH_3COOH$ in a yield of 71%.

On the other hand, $9.12 \times 10^{-3}$ kg (9.12 g) of benzyloxycarbonyl-Ser-$NHNH_2$ was suspended in $5 \times 10^{-6}m^3$ (5.0 ml) of dimethylformamide. While cooling to −50 to −40°C, $1.8 \times 10^{-5}m^3$ (18 ml) of a 6N hydrogen chloride solution in dioxane was added to this suspension. $5.4 \times 10^{-6}M^3$ (5.4 ml) of isoamyl nitrite was added to this mixture. After further addition of $1.52 \times 10^{-5}m^3$ (15.2 ml) of triethylamine, the thus prepared mixture was added to a mixture solvent of $3.6 \times 10^{-5}m^3$ (36 ml) of di-

methylsulfoxide and $1.8 \times 10^{-5}m^3$ (18 ml) of dimethylformamide containing $1.09 \times 10^{-5}m^3$ (10.9 ml) of triethylamine and $1.1 \times 10^{-2}$ kg (11.0 g) of the above-mentined H-Asn-Tyr-OH.$CH_3COOH$. This reaction mixture was stirred overnight at a temperature of −5 to 0°C. Then, after evaporating off the solvents from the reaction mixture under reduced pressure, water was added to the mixture and the pH of the mixture was adjusted to 8 by addition of sodium hydrogen carbonate. The thus adjusted mixture was extracted with ethyl acetate. On adding sodium chloride to the aqueous layer and acidifying this layer with citric acid, a gel-like substance was formed. The substance was collected by filtration, washed with water and dried. $1.42 \times 10^{-2}$Kg (14.2 g) of the dried substance were obtained. After dissolving the substance in a mixed solvent of $1.8 \times 10^{-4}$ $m^3$ (180 ml) of methanol, $2.6 \times 10^{-4}$ $m^3$ (260 ml) of water and $9 \times 10^{-6}m^3$ (9 ml) of acetic acid, the substance was subjected to catalytic hydrogenation in the presence of $5 \times 10^{-4}$Kg (0.5 g) of palladium black at ambient temperature and pressure. After the reaction was over, the reaction mixture was filtered. The filtrate was condensed under reduced pressure. On recrystallizing the residue from a mixture of water and ethanol, H-Ser-Asn-Tyr-OH was obtained in a yield of 62%. The product melted at a temperature higher than 200°C.

(2) Synthesis of H-Gly-Gly-Ser-Asn-Tyr-OH

H-Ser-Asn-Tyr-OH obtained as above was condensed with benzyloxycarbonyl-Gly-Gly-$NHNH_2$ according to the azide method to form benzyloxycarbonyl-Gly-Gly-Ser-Asn-Tyr-OH. This product was subjected to catalytic hydrogenation in the presence of palladium black to obtain the desired product, H-Gly-Gly-Ser-Asn-Tyr-OH.

(3) Synthesis of H-Lys($N^{\epsilon}$-tosyl)-Ser-Gln-Gly-Gly-Ser-Asn-Tyr-OH

H-Gly-Gly-Ser-Asn-Tyr-OH obtained as above was condensed with benzyloxycarbonyl-Lys($N^{\epsilon}$-tosyl)-Ser-Gln-NH.$NH_2$ by the azide method. The condensate was subjected to catalytic hydrogenation is the presence of palladium black to form the desired product in a yield of 42%. The amino acid composition of the product was:
$NH_3$—2.19; Lys—0.38; Gly—2.00; Glu—0.97, Ser—1.81; Tyr—0.99 and Asp—1.00.

(4) Synthesis of H-Ala-Lys($N^{\epsilon}$-tosyl)-Ser-Gln-Gly-Gly-Ser-Asn-Tyr-OH

$2.2 \times 10^{-7}$ $M^3$ (0.22 ml) of N-methylmorpholine was added to a solution of $2.0 \times 10^{-3}$ kg (2.0 g) of the above-mentioned H-Lys($N^{\epsilon}$-tosyl)-Ser-Gln-Gly-Gly-Ser-Asn-Tyr-OH in $1.5 \times 10^{-5}$ $m^3$ (15 ml) of dimethylsulfoxide. A solution of $6.85 \times 10^{-4}$ kg (685 mg) of benzyloxycarbonylalanine N-hydroxysuccinimide ester in $5 \times 10^{-6}$ $m^3$ (5 ml) of dimethylsulfoxide was added to this mixture. The resultant mixture was stirred overnight at room temperature. The reaction mixture was then diluted with $2 \times 10^{-4}$ $m^3$ (200 ml) of water and citric acid was added to form a gel-like precipitate, which was collected by filtration, washed with water, ethanol, ethyl acetate and ether in that order, dried and then recrystallized from a mixture of dimethyl-formamide and ethanol. After dissolving the crystals in an aqueous 50% acetic acid solution, this solution was subjected to catalytic hydrogenation in the presence of palladium black. After filtration of the reaction mixture and evaporating off the solvent from the filtrate, the thus separated substance was recrystallized from an aqueous ethanolic solution, washed with ethanol and ether and dried to obtain the desired product in a yield of 64%. The thus obtained product showed the following amino acid composition: $NH_3$—2.01; Ala—1.05; Lys—0.30; Gly—2.08, Glu—0.96; Ser—1.71; Tyr—0.94 and Asp—1.00.

(5) The last step: Synthesis of pGlu-Ala-Lys-Ser-Gln-Gly-Gly-Ser-Asn-Tyr-OH

$10^{-7}$ $M^3$ (0.1 ml) of N-methylmorpholine and then $3.55 \times 10^{-4}$ kg (355 mg) of benzyloxycarbonyl-pGlu N-hydroxysuccinimide ester were added to a solution of $9.5 \times 10^{-4}$ kg (950 mg) of the abovementioned H-Ala-Lys($N_{\epsilon}$-tosyl)-Ser-Gln-Gly-Gly-Ser-Asn-Tyr-OH in 15 ml of dimethylsulfoxide. This mixture was stirred overnight at room temperature. After the reaction was over, $3 \times 10^{-4}$ $m^3$ (300 ml) of water was added to the reaction mixture. This mixture was made acidic by the addition of citric acid and cooled with water. A gel-like product separated out and was collected by filtration, washed with a dilute aqueous citric acid solution, water, ethanol, ethyl acetate and ether in that order and dried. The dried substance was subjected to catalytic hydrogenation in the presence of 10% palladium on carbon. The hydrogenated material was dissolved in $2 \times 10^{-4}$ $m^3$ (200 ml) of liquid ammonia. Metallic sodium was added to the solution little by little, and at the time point when the solution was tinged blue $10^{-3}$ kg (1 g) of ammonium chloride was added to neutralise the solution. After evaporating off ammonia, the residue was dissolved in $8 \times 10^{-6}$ $m^3$ (8 ml) of water and extracted with ether. The aqueous layer was separated and subjected to treatment with Biogel P—2 for purification using water as an eluant. The purified product obtained in a yield of 46% showed the following amino acid composition: $NH_3$—3.51; Ala—1.07; Lys—1.04; Gly—2.06; Glu—2.05; Ser—1.90; Tyr—0.98 and Asp—1.00.

Example 3: Synthesis of Compound No 3:

(1) Synthesis of H-Tyr-Gln-Ala-Lys-Ser-Gln-Gly-Gly-Ser-Asn-OH

$1.1 \times 10^{-6}M^3$ (1.1 ml) of a 6N solution of hydrogen chloride in dioxane was added dropwise to a

solution of 1.006 × $10^{-3}$ kg (1.006 g) of benzyloxycarbonyl-Tyr-Gln hydrazide in 4 × $10^{-5}$ $m^3$ (40 ml) of dimethylformamide, while cooling. Then 4 × $10^{-7}$$m^3$ (0.4 ml) of isoamyl nitrite and 9.3 × $10^{-7}$ $m^3$ (0.93 ml) of triethylamine were added to the mixture. A solution of 1.696 × $10^{-3}$kg (1.696 g) of H-Ala-Lys($N^{\epsilon}$-t-butoxycarbonyl)-Ser-Gln-Gly-Gly-Ser-Asn-OH in a mixed solvent of 2 × $10^{-5}$ $m^3$ (20 ml) of dimethylformamide and 2 × $10^{-5}$ $m^3$ (20 ml) of water and 2.8 × $10^{-7}$ $m^3$ (0.28 ml) of triethylamine were added. This mixture was stirred for one hour at −15°C and then for 3 days at 0 to 4°C. After the reaction was over, the solvents were evaporated off from the reaction mixture under reduced pressure. The residue was dissolved in water containing a few drops of acetic acid. After again evaporating off the solvent under reduced pressure ether was added to the residue to cause crystals to separate out. The crystals were washed with ethyl acetate, dried and recrystallized from a mixed solvent of dimethylformamide and ethanol. After subjecting the crystals to catalytic hydrogenation in the presence of palladium black, the hydrogenated material was dissolved in $10^{-5}$ $m^3$ (10 ml) of trifluoroacetic acid containing $10^{-6}$ $m^3$ (1 ml) of anisol. The solution was stirred for one hour at room temperature.

After evaporating off the solvent from the solution, ether was added to the residue to cause crystals to separate out. These crystals were washed with ether and dried. The dried crystals were dissolved in water and after passing the solution through a column of an anion-exchange resin (IRA—411), the solution was purified by Biogel P—2 using water as an eluant. The thus prepared desired compound showed the following amino acid composition: $NH_3$—3.15; Ala—1.01; Lys—1.01; Gly—1.98; Glu—2.02, Ser—1.80; Tyr—1.00 and Asp—1.00.

Example 4: Rosette Test

A mouse 10 weeks after its birth was subjected to extirpation of its thymus. 10 days after extirpation, when the slenic cells of the mouse had still not differentiated and had few theta-antigens on their surface otherwise induced by the influence of serum thymic factor, the spleen of the mouse was extirpated. The cells of the thus extirpation spleen were subjected to the following tests to analyse the formation of "rosettes" under a microscope:

(1) Test No 1:

The splenic cells were incubated with sheep red blood cells (SRBC) in the presence of azathiopurine at a concentration of 10 micrograms/ml. The number of rosette-like aggregates formed by the combination of T-cells among the splenic cells and SRBC was counted under a microscope. The number of the rosette-like aggregates (so-called "rosettes" in this type of experiment) was, on average, $10^7$/$m^3$ (10/ml).

(2) Test No 2:

The splenic cells were incubated with SRBC in the presence of azathiopurine at the same concentration as in (1) and further in the presence of each of the respective peptides synthesized in Examples 1 to 3 and a specimen of STF collected from an adult mouse. The formation of "rosettes" in each incubate was observed as in (1) under a microscope. The number of "rosettes" was on average, 4.2: 4.0: 3.5: and 5.0; respectively.

(3) Evaluation of rate of inhibiting rosette-formation:

In the asbsence of STF, the splenic cells of the adult thymectomized mice form "rosettes" by combining with SRBC (more than two SRBC combining with one splenic cell) regardless of the presence or absence of azathiopurine at the concentration in (1) when incubated with SRBC.

However, in the presence of STF or a substance with STF-like activity, the theta-antigen is induced on the cell surface of T-cells. As a result, azathiopurine inhibits the formation of "rosettes" between SRBC and the splenic cells having the theta-antigen on their surface at the same concentration as in (1) or (2).

Thus, the activity of STF or a substance having STF-like activity in preventing the formation of "rosettes" is represented and calculated by the following fomula:

$$\text{Activity} = \frac{N_0 - N_1}{N_0} \times 100$$

wherein $N_0$ means the number of rosettes formed in the case where no STF or a substance having STF-like activity was present and azathiopurine was present, and

$N_1$ means the number of rosettes formed where

(1) STF and azathiopurine were present and

(2) a subtance having STF-like activity and azothiopurine were present, respectively.

Using the data obtained in Test No. 2 in the abovementioned formula, the activity of the specimen of STF, and the activities of the peptides synthesized in the Examples according to the present invention were obtained, the activities being:

| Substance | Activity of preventing "rosette" formation |
|---|---|
| STF | 50% |
| Compound No 1 | 58% |
| Compound No 2 | 60% |
| Compound No 3 | 65% |

Example 5: Radio-labelling of the synthesized peptide

$10^{-8}$ $M^3$ (10 microlitres) of aqueous 0.1M acetic acid solution containing $5 \times 10^{-4}$ kg (5 micrograms) of Compund No 3 of the present invention, H-Tyr-Gln-Ala-Lys-Ser-Gln-Gly-Gly-Ser-Asn-OH, $10^{-8}$ $m^3$ (10 microlitres) of water containing $10^{-3}$ Ci (1 mCi) of sodium 125 iodide, and $2 \times 10^{-8}$ kg (20 micrograms) of chloramin T were added in this order to $2 \times 10^{-8}$ $m^3$ (20 microlitres) of aqueous 1.0M phosphate buffer solution of pH 7.3.

After shaking the mixture for 20 sec, $5 \times 10^{-8}$ $m^3$ (50 microlitres) of water containing $10^{-7}$ kg (100 micrograms) of sodium hydrogen metasulfite and $10^{-8}$ $m^3$ (10 microlitres) of aqueous 10% potassium iodide solution were added to the mixture.

The thus formed mixture was subjected to gel chromatography using Sephadex G—10 to purify the product. Aqueous 0.1M acetic acid solution was employed as an eluant. The weight of the thus obtained labelled product was $4 \times 10^{-9}$ kg (4.0 microgram) containing 46% of the radioactive iodine originally in the radioactive sodium iodide used for the labelling. The specific radioactivity of the thus labelled peptide, Compound No 3 of the present invention, due to $^{125}I$, was $1.15 \times 10^{-1}$ Ci/kg (115 mCi/mg).

**Claims**

1. A peptide with a primary structure represented by the general formula (I):

X-Ser-Gln-Gly-Gly-Ser-Asn-Y (I)

wherein

(1) X represents H-Tyr-Gln-Ala-Lys and Y represents a hydroxyl group,

(2) X represents pGlu-Ala-Lys($N^{\varepsilon}$-Tyr-H) and Y represents a hydroxyl group, or

(3) X represents pGlu-Ala-Lys and Y represents a Tyr-OH group, and

(a) -Lys($N^{\varepsilon}$-Tyr-H) means that the amino group at the ε-position of the side chain of lysine is bonded to tryosine via a peptide bond, and (b) pGlu represents a group which has been formed by removal of a hydroxy group from the carboxy group of pyroglutamic acid.

2. A process for producing a peptide with a primary structure of formula (I) as defined in claim 1, which process comprises condensing the appropriate amino acid(s) and/or peptide(s) by the azide, acid anhydride or active ester method in order to obtain the peptide with the desired primary structure.

3. A process for producing a peptide with a primary structure of formula (I) as defined in claim 1, which process comprises bonding a predetermined amino acid or peptide to the N-end of H-Asn-OH, H-Asn-Tyr-OH or a peptide of the appropriate primary structure and terminating in -Asn-OH or -Asn-Tyr-OH at its C-end without protecting the carboxyl group at the C-end- of said H-Asn-OH, H-Asn-Tyr-OH or peptide terminating in -Asn-OH or -Asn-Tyr-OH at its C-end, and as desired elongating the resulting peptide chain from the N-end by the bonding of further predetermined amino acid(s) and/or peptide(s), to obtain the desired peptide.

4. A process according to claim 3 wherein the carboxyl group at the C-end remains unprotected during said elongation with further predetermined amino acid(s) and/or peptide(s).

5. A peptide as claimed in claim 1, or which has been produced by a process as claimed in any one of claims 2 to 4, labelled with radioactive iodine.

6. Use of a radioactively labelled peptide as claimed in claim 5 in the radioimmunoassay of serum thymic factor.

**Revendications**

1. Peptide ayant une structure primaire représentée par la formule générale (I):

X-Ser-Gln-Gly-Gly-Ser-Asn-Y (I)

dans laquelle:

(1) X représente H-Tyr-Gln-Ala-Lys; et

Y represésente un groupe hydroxyle;

(2) X représente pGlu-Ala-Lys($N^{\varepsilon}$-Tyr-H); et
Y représente un groupe hydroxyle; ou
(3) X représente pGlu-Ala-Lys; et
Y représente un groupe Tyr-OH; et
(a) -Lys($N^{\varepsilon}$-Tyr-H) signifie que le groupe aminé en position-$\varepsilon$ de la chaîne latérale de lysine est lié à la tyzosine par une liaison peptidique; et
(b) pGlu représente un groupe qui est formé par enlèvement d'un groupe hydroxy du groupe carboxy de l'acide pyroglutamique.

2. Procédé de préparation d'un peptide ayant une structure primaire de formule (I) telle que définie dans la revendication 1, procédé consistant à condenser 1'(les) acide(s) amine(s) approprié(s) et/ou peptide(s) par la méthode à l'azoture, la méthode à l'anhydride acide ou la méthode à l'ester actif en vue d'obtenir le peptide ayant la structure primaire désirée.

3. Procédé de préparation d'un peptide ayant une structure primaire de formule (I) telle que décrite dans la revendication 1, procédé consistant à lier un amino-acide ou peptide prédéterminé à l'extrémité-N de H-Asn-OH, H-Asn-Tyr-OH ou un peptide de la structure primaire approprié terminant par Asn-OH ou -Asn-Tyr-OH à son extrémité -C sans protéger le groupe carboxyle à l'extrémite -C dudit H-Asn-OH, H-Asn-Tyr-OH ou un peptide terminant par -Asn-OH ou -Asn-Tyr-OH à son extrémité -C, et si on le désire à allonger la chaîne peptidique qui en résulte à partir de l'extrémité -N par liaison d'autres amino-acide(s) et/ou peptide(s) prédéterminé(s) pour obtenir le peptide désiré.

4. Procédé suivant la revendication 2, dans lequel le groupe carboxyle à l'extrémité -C reste sans protection pendant ledit allongement avec les amino-acide(s) et/ou peptide(s) prédéterminé(s).

5. Peptide suivant la revendication 1, ou ayant été produit par un procédé tel que revendiqué dans l'une quelconque des revendications 2 à 4, marqué par de l'iode radio-actif.

6. Utilisation d'un peptide marqué radio-activement tel que revendiqué dans la revendication 5 dans l'essai radio-immunologique du facteur thymique sérique.

**Patentansprüche**

1. Peptid mit einer primären Struktur der allgemeinen Formel (I):

X-Ser-Gln-Gly-Gly-Ser-Asn-Y (I)

worin
(1) X für H-Tyr-Gln-Ala-Lys steht und Y eine Hydroxylgruppe bedeutet,
(2) X pGlu-Ala-Lys($N^{\varepsilon}$-Tyr-H) ist und Y eine Hydroxylgruppe darstellt oder
(3) X pGlu-Ala-Lys versinnbildlicht und Y eine Tyr-OH-Gruppe ist und
(a) -Lys($N^{\varepsilon}$-Tyr-H) bedeutet, daß die Aminogruppe in der $\varepsilon$-Stellung der Nebenkette des Lysins mit Tyrosin über eine Peptidbindung verbunden ist, und
(b) pGlu eine Gruppe bedeutet, die durch Entfernung einer Hydroxygruppe von der Carboxygruppe der Pyroglutaminsäure gebildet worden ist.

2. Verfahren zur Herstellung eines Peptids mit einer Primären Struktur der Formel (I) gemäß Anspruch 1, dadurch gekennzeichnet, daß die entsprechende Aminosäure(n) und/oder Peptid(e) nach der Azid-, Säureanhydrid- oder aktiven Estermethode zur Gewinnung des Peptids mit der gewünschten primären Struktur kondensiert wird (werden).

3. Verfahren zur Herstellung eines Peptids mit einer primären Struktur der Formel (I) gemäß Anspruch 1, dadurch gekennzeichnet, daß eine vorherbestimmte Aminosäure oder ein Peptid mit dem N-Ende, von H-Asn-OH, H-Asn-Tyr-OH oder eines Peptids der entsprechenden primären Struktur, das in -Asn-OH oder -Asn-Tyr-OH an dem C-Ende endet, ohne Schützen der Carboxylgruppe an dem C-Ende von H-Asn-OH, H-Asn-Tyr-OH oder des Peptids, das in -Asn-OH oder -Asn-Tyr-OH an dem C-Ende endet, verbunden wird und gegebenenfalls die erhaltene Peptidkette von dem N-Ende an durch Anbinden einer weiteren vorherbestimmten Aminosäure oder weiterer Aminosäuren und/oder eines Peptids oder von Peptiden zur Gewinnung des gewünschten Peptids verlängert wird.

4. Verfahren nach Anspruch 3, dadurch gekennzeichnet, daß die Carboxylgruppe an dem C-Ende während der Verlängerung mit einer weiteren vorherbestimmten Aminosäure oder mit weiteren Aminosäuren und/oder eines Peptids oder Peptiden ungeschützt bleibt.

5. Peptid nach Anspruch 1 oder Peptid, das nach einem Verfahren gemäß einem der Ansprüche 2 bis 4 erhalten worden ist und mit readioaktivem Jod markiert ist.

6. Verwendung eines radioaktiv markierten Peptids gemäß Anspruch 5 bei der Radioimmunoassay von Serumthymusfaktor.